# EUROPÄISCHE PATENTSCHRIFT

(11) **EP 3 296 959 B1**
(45) Veröffentlichungstag und Bekanntmachung des Hinweises auf die Patenterteilung: **01.09.2021**
(21) Anmeldenummer: 17190919.5
(22) Anmeldetag: 13.09.2017
(51) Int. Cl.: H04N 13/246, H04N 13/189, G06T 7/80

(54) **JUSTIERSYSTEM**
ADJUSTING SYSTEM
SYSTÈME D'ALIGNEMENT

(30) Priorität: 16.09.2016 DE 102016217792
(43) Veröffentlichungstag der Anmeldung: 21.03.2018
(73) Patentinhaber: XION GmbH, 13127 Berlin (DE)
(72) Erfinder: Müller, Holger, 16548 Glienicke-Nordbahn (DE)
(74) Vertreter: Eisenführ Speiser

(56) Entgegenhaltungen:
- DE-A1- 19 536 297
- JP-A- 2009 258 427
- US-A1- 2010 245 541
- THOMAS STEHLE ET AL: "Dynamic Distortion Correction for Endoscopy Systems with Exchangeable Optics", BILDVERARBEITUNG FÜR DIE MEDIZIN, 1. Januar 2009 (2009-01-01), Seiten 142-146, XP055045061, DOI: 10.1007/978-3-540-93860-6_29
- Christian Wengert ET AL: "Fully Automatic Endoscope Calibration for Intraoperative Use" In: "Bildverarbeitung für die Medizin", 1. Januar 2006 (2006-01-01), Springer, XP055349662, ISBN: 978-3-540-32136-1 Seiten 419-423, DOI: 10.1007/3-540-32137-3_85, * Zusammenfassung *
- MARIUS SCHWALBE ET AL: "Design and Implementation of a Laparoscope Calibration Method for Augmented Reality Navigation", TAGUNGSBAND DER 14. JAHRESTAGUNG DER DEUTSCHEN GESELLSCHAFT FÜR COMPUTER- UND ROBOTERASSISTIERTE CHIRURGIE E.V, 1. September 2015 (2015-09-01), XP055442742, ISBN: 978-3-00-050359-7
- MELO R ET AL: "A New Solution for Camera Calibration and Real-Time Image Distortion Correction in Medical Endoscopy Initial Technical Evaluation", IEEE TRANSACTIONS ON BIOMEDICAL ENGINEERING, IEEE SERVICE CENTER, PISCATAWAY, NJ, USA, Bd. 59, Nr. 3, 1. März 2012 (2012-03-01), Seiten 634-644, XP011489985, ISSN: 0018-9294, DOI: 10.1109/TBME.2011.2177268

## Beschreibung

Die Erfindung betrifft ein Verfahren zum Justieren eines stereoskopischen optischen Systems. Weiterhin betrifft die Erfindung ein Justiersystem zur Verwendung in einem stereoskopischen optischen System, die Verwendung eines Justiersystems, ein medizinisches stereoskopisches Gerät, insbesondere ein Stereoendoskop und ein starres Endoskop für die Anwendung mit Navigationssystemen, und ein Computer-Programm.

Bei optischen Geräten, wie beispielsweise starren Endoskopen und auch anderen medizinischen optischen Geräten mit optischer Bildübertragung, kann es zu deutlichen Abweichungen der Bildposition von dem Ideal eines vollständig zentrierten optischen Systems kommen. Ursache sind Fertigungstoleranzen, thermische Ausdehnung oder eine mechanische Durchbiegung des optischen Geräts. So kann eine Durchbiegung bei derartigen bildübertragenden optischen Systemen wegen der hohen Anzahl optischer Elemente, die beispielsweise in Endoskopen vorliegt, zu deutlichen Veränderungen eines ausgegebenen Bildes führen.

In der Stereoskopie ist eine relative Justierung der stereoskopischen Halbbilder besonders wichtig. Andernfalls wird der stereoskopische Bildeindruck verschlechtert und eine Ermüdung des Betrachters tritt auf.

Dokument US 8,223,193 B2 beschreibt eine Kalibriervorrichtung, die eine Schnittstelle zum festen Anordnen an einem endoskopischen Bildgebungssystem, ein mit der Schnittstelle verbundenes Zielobjekt im Sichtfeld des Bildgebungssystem, und einen Prozessor aufweist. Der Prozessor ist ausgebildet, Markierungen auf ersten und zweiten Bildern aus einer ersten und zweiten Anordnung von Bildgebungssystem und Zielobjekt zueinander zu identifizieren. Hierbei werden die Anordnung und die identifizierten Markierungen genutzt um Kalibrierungsdaten für das endoskopischen Bildgebungssystem zu bestimmen.

Aus US 2010/245541 A1 ist das Kalibrieren einer endoskopischen Kamera bekannt.

Aus THOMAS STEHLE ET AL: "Dynamic Distortion Correction for Endoscopy Systems with Exchangeable Optics", BILDVERARBEITUNG FÜR DIE MEDIZIN, 1. Januar 2009 (2009-01 -01 ), Seiten 142-146, ist eine dynamische Korrektur der Verzeichnung bei Endoskopen mit Wechseloptiken bekannt.

Ziel der Erfindung ist es ein verbessertes Verfahren zum Justieren eines stereoskopischen optischen Systems zu schaffen.

Erfindungsgemäß wird dieses Ziel gemäß einem ersten Aspekt der Erfindung durch ein Verfahren zum Justieren eines optischen Systems, insbesondere eines stereoskopischen optischen Systems erreicht. Das Verfahren umfasst erste, statische Justierschritte und zweite, dynamische Justierschritte. Die ersten, statischen Justierschritte weisen die folgenden Schritte auf:
- Anordnen eines vorbestimmten Referenzobjekts mit bekannten sichtbaren Referenzobjekt-Merkmalen in einem durch das optische System erfassten Gesichtsfeld;
- Erfassen eines durch das optische System bereitgestellten Bildes des vorbestimmten Referenzobjekts oder bei einem stereoskopischen optischen System eines ersten und/oder zweiten Halbbildes des vorbestimmten Referenzobjekts durch ein Sensorsystem, insbesondere in Form von Referenzobjekt-Daten, wobei das erste und das zweite Halbbild auf einer Abbildungsfläche des Sensorsystems abgebildet werden;
- Bestimmen einer Positionsinformation mindestens zweier in dem Bild oder in dem ersten und/oder zweiten Halbbild abgebildeter sichtbarer Referenzobjekt-Merkmale des vorbestimmten Referenzobjekts;
- Ermitteln einer optischen Verzeichnung des Bildes oder des ersten und/oder zweiten Halbbildes aus der Positionsinformation der mindestens zwei sichtbaren ReferenzobjektMerkmale;
- Justieren eines mit dem Sensorsystem zumindest indirekt verbundenen Bildprozessors, durch Einstellen einer geometrischen Entzerrung entsprechend der ermittelten optischen Verzeichnung.

Weiterhin weist das Verfahren während eines Betriebs des optischen Systems zum Erfassen eines zu untersuchenden Objekts die folgenden zweiten, dynamischen Justierschritte auf:
- Anordnen des zu untersuchenden Objekts im durch das optische System erfassten Gesichtsfeld;
- Erfassen von Lagedaten, welche eine durch mindestens einen Referenzpunkt bestimmte Lageabweichung eines durch das optische System bereitgestellten Bildes des zu untersuchenden Objekts gegenüber erfassten Lagedaten früherer Zeitpunkte zur Ermittlung einer Beschleunigungs-information des optischen Systems oder bei einem stereoskopischen optischen System einer gegenseitigen Lage eines ersten und zweiten Halbbildes des zu untersuchenden Objekts durch das Sensorsystem enthalten; und
- Justieren des Bildprozessors entsprechend der erfassten Lageabweichung durch Einstellen einer entsprechenden Verschiebung des Bildes oder des ersten und zweiten Halbbildes gegeneinander durch den Bildprozessor.

Zunächst wird somit eine optische Verzeichnung ermittelt und ein Bildprozessor so justiert, dass er die ermittelte optische Verzeichnung durch geometrische Entzerrung kompensiert. Dieser Schritt kann statisch mit Hilfe eines Referenzobjektes erfolgen und muss nicht ständig wiederholt werden, da sich die optische Verzeichnung des optischen Systems typischerweise während des Betriebs nicht ändert.

Anschließend kann eine Lageabweichung erfasst und der Bildprozessor so justiert, dass er die ermittelte Lageabweichung durch Verschieben des Bildes oder der ersten und zweiten Halbbilder kompensiert. Diese Kompensation der Lageabweichung kann während des regulären Betriebes wiederholt - also dynamisch - erfolgen, um während des Betriebs auftretende Lageabweichungen ad hoc zu kompensieren.

Ein Aspekt ist somit die Aufteilung des Verfahrens in statische und dynamische Justierschritte.

Ein hiervon auch unabhängig zu verwirklichender Aspekt ist derjenige, dass zunächst die optische Verzeichnung kompensiert wird und anschließend eine mögliche Lageabweichung.

Besondere Vorteile ergeben sich, wenn beide Aspekte wie vorstehend geschildert kombiniert werden, und zwar insbesondere beim Justieren eines stereoskopischen optischen Systems, weil dort die Kompensation der Lageabweichung der Halbbilder von besonderer Relevanz ist, um Doppelbilder zu vermeiden.

Durch das erfindungsgemäße Verfahren wird vorteilhaft eine verbesserte Justage und eine Verzeichnungskorrektur des optischen Systems erreicht. Hierbei wird das Bild oder im Falle eines stereoskopischen optischen Systems werden die beiden stereoskopischen Halbbilder entzerrt und entsprechend einer Lageabweichung verschoben. Insbesondere wird im besonders vorteilhaften stereoskopischen Fall sowohl eine Verzeichnungskorrektur, als auch eine Korrektur zwischen der gegenseitigen Lage des ersten und zweiten Halbbildes untereinander vorgenommen. Unter Verzeichnung wird hierbei die Abhängigkeit des Abbildungsmaßstabes eines Abbildungskanals des optischen Systems von einem Bildort und insbesondere von einem Feldwinkel verstanden.

Die Anzahl der zum Justieren verwendeten Referenzobjektmerkmale muss mindestens zwei sein. Es kann sich hierbei beispielsweise um die Krümmung und Position einer geraden Linie auf einer Messtafel handeln. Zur Erhöhung einer Justiergenauigkeit kann eine größere Zahl von Referenzobjektmerkmalen gewählt werden.

Vorteilhaft ist, dass das Verfahren sowohl statische Justierschritte, die mit dem Referenzobjekt durchgeführt werden, als auch dynamische Justierschritte während des Betriebs des optischen Systems zum Erfassen eines zu untersuchenden Objekts aufweist. Hierdurch wird eine Korrektur während des Betriebs, also beispielsweise während einer Operation ermöglicht. Außerdem wird die Korrektur während des Betriebs beschleunigt, da die während der statischen Justierschritte korrigierte Verzeichnung nicht mehr dynamisch korrigiert werden muss, wodurch die Verarbeitungszeit der dynamischen Justierschritte verringert wird. Dadurch, dass während der dynamischen Justierschritte sehr wenig Parameter bestimmt werden müssen, kann das erfindungsgemäße Verfahren die Zuverlässigkeit und Genauigkeit der Bildverarbeitung bei geringen Datenraten erhöhen.

Insbesondere kann auch mit einer geringen Anzahl an zu messenden Bildfeldpunkten des zu untersuchenden Objekts eine hohe Genauigkeit der Justage erreicht werden.

Die statischen Justierschritte müssen deutlich seltener ausgeführt werden als die dynamischen Justierschritte, ohne dass dadurch eine Qualität der Justage des optischen Systems deutlich beeinträchtigt ist. Daher führt das erfindungsgemäße System auch zu einer geringen Gesamtjustagedauer und entsprechend zu einem geringen Aufwand der Justage.

Vorteilhaft ist weiterhin, dass das Verfahren weitgehend automatisiert durchgeführt werden kann, so dass es von Nutzern des optischen Systems, also beispielsweise medizinischem Fachpersonal, auch ohne Spezialkenntnisse auf dem Gebiet der Kamerajustierung oder -kalibrierung schnell, sicher und unkompliziert ausgeführt werden kann.

Da das Verfahren sich auf Schritte der Bildverarbeitung beschränkt, kann es für viele stereoskopische optische Systeme genutzt werden. Folglich weist es viele mögliche stereoskopische Anwendungsbereiche auf, die nicht auf die im Folgenden betonte Anwendung für Stereoendoskope beschränkt ist.

Unter Gesichtsfeld wird im Folgenden der Raum verstanden, in dem Objekte vom optischen System erfasst werden und zur Abbildung in ein Auge oder auf einen Bildsensor gelangen können.

Das Einstellen einer geometrischen Entzerrung erfolgt durch eine elektronische oder optische Veränderung einer geometrischen Lage von Bildpunkten zueinander entsprechend der ermittelten optischen Verzeichnung.

Ein Referenzobjekt kann im Rahmen der Erfindung jedes Objekt mit bekannten sichtbaren Referenzobjekt-Merkmalen sein. Insbesondere kann eine bedruckte Scheibe mit Markierung, eine bedruckte Folie, ein strukturierter Hintergrund oder ein vorbestimmt angeordneter geometrischer Körper vorteilhaft als Referenzobjekt genutzt werden.

Nachfolgend werden bevorzugte Ausführungsformen des erfindungsgemäßen Verfahrens genannt.

Der mindestens eine Referenzpunkt aus dem die Lagedaten bestimmt werden, ist in einer bevorzugten Ausführungsform ein Bildpunkt des Bildes oder des ersten und/oder zweiten Halbbildes. In anderen Ausführungsformen ist der mindestens eine Referenzpunkt eine geometrische Abstandsangabe oder ein mechanischer Druck, welche von mechanischen Deformationssensoren des optischen Systems bestimmt werden und die das Vorliegen einer Deformation des optischen Systems sowie ein Maß für die Deformation implizieren. In einer Variante verwendet das optische System hierfür einen optischen Referenzstrahl, der innerhalb des Strahlengangs des optischen Systems verläuft und das optische System dabei nicht in Richtung des zu untersuchenden Objekts verlässt, Dies wird in der Variante beispielsweise durch einen Spiegel auf einer zu dem Objekt gewandten Objektseite des optischen Systems realisiert, der mindestens in einem Wellenlängenbereich mindestens teilweise reflektierend für den optischen Referenzstrahl ist.

In einer bevorzugten Ausführungsform beschreiben die Lagedaten einen geometrischen Abstand. In einer weiteren Ausführungsform beschreiben die Lagedaten eine Beschleunigungsinformation, wobei hierbei eine Position aus Positionsdaten des mindestens einen Referenzpunkts zu mindestens zwei verschiedenen Zeitpunkten bestimmt wird. Dies ist besonders vorteilhaft für das Justieren des Bildprozessors bei Vorliegen einer regelmäßigen Bewegung des optischen Systems und/oder des zu untersuchenden Objekts, wie beispielsweise bei einem Wackeln des optischen Systems. Hierbei kann der Bildprozessor besonders genau justiert werden, da auch Lagedaten zu einem anderen Zeitpunkt für das Justieren berücksichtigt werden.

In einer weiteren Ausführungsform werden die Lagedaten über ein Navigationssystem erfasst. Das Navigationssystem ist dabei ausgebildet, eine Position und/oder Ausrichtung eines zum Objekt gewandten distalen Teils des optischen Systems zu überwachen. Aufbau und Funktionsweise derartiger Navigationssysteme sind insbesondere für Anwendungen im medizinischen Bereich und dabei insbesondere für Anwendungen im Bereich der Endoskopie bereits allgemein bekannt.

Die Erfassung der Lageabweichung ist in einer Ausführungsform für ein stereoskopisches endoskopisches System durch folgenden Ablauf realisiert:
- Anordnen des zu untersuchenden Objekts mit einem vorbestimmten sichtbaren Untersuchungsobjekt-Merkmal im durch das optische System erfassten Gesichtsfeld;
- Erfassen eines durch das optische System bereitgestellten ersten und zweiten Halbbildes des zu untersuchenden Objekts durch das Sensorsystem, insbesondere das Erfassen von Untersuchungsobjekt-Daten;
- Bestimmen einer ersten vertikalen und/oder horizontalen Bildkoordinate des Untersuchungsobjekt-Merkmals in dem ersten Halbbild und einer zweiten entsprechend vertikalen und/oder horizontalen Bildkoordinate des Untersuchungsobjekt-Merkmals in dem zweiten Halbbild,
   - wobei eine etwaige horizontale Bildkoordinate bezüglich einer horizontalen Bildachse bestimmt wird, die innerhalb einer Bildebene des stereoskopischen optischen Systems liegt und beide optische Achsen des stereoskopischen optischen Systems schneidet, und
   - wobei eine etwaige vertikale Bildkoordinate bezüglich einer vertikalen Bildachse bestimmt wird, die innerhalb der Bildebene des optischen Systems und senkrecht zur horizontalen Bildachse liegt.

Die jeweiligen vertikalen oder horizontalen Bildkoordinaten können im Rahmen der Erfindung entlang der vertikalen oder horizontalen Bildachse bestimmt werden, aber auch entlang einer Richtung, die im Wesentlichen entlang der vertikalen oder horizontalen Bildachse orientiert ist, jedoch nicht mit dieser übereinstimmt.

Weiterhin können die vertikalen oder horizontalen Bildkoordinaten direkt aus einer Abbildung des zu untersuchenden Objekts oder durch Verarbeitung der Untersuchungsobjekt-Daten als virtuelle Bildkoordinaten gewonnen werden.

Besonders vorteilhaft ist das Nutzen von vertikalen Bildkoordinaten. Bei vielen Anwendungen von stereoskopischen optischen Systemen ist ein Abstand zwischen zu untersuchendem Objekt und optischem System nicht bekannt, so dass ein Versatz zwischen horizontalen Koordinaten eines Untersuchungsobjekt-Merkmals aufgrund einer Parallaxe gewollt ist und entsprechend nicht korrigiert werden sollte. Ein Versatz der vertikalen Bildkoordinaten stellt hingegen in den meisten möglichen Anwendungen von stereoskopischen optischen Systemen einen unerwünschten Fehler dar. Daher werden in bevorzugten Ausführungsformen des erfindungsgemäßen Verfahrens mindestens die vertikalen Bildkoordinaten des Untersuchungsobjekt-Merkmals zum Einstellen einer entsprechenden Verschiebung des ersten und zweiten Halbbildes genutzt.

In einer Ausführungsform weist das Verfahren auch ein Projizieren von Messmarken auf das zu untersuchenden Objekt und/oder das Bild oder bei einem stereoskopischen optischen System auf mindestens eines der Halbbilder auf.

In einer weiteren Ausführungsform ist das Sensorsystem mit Sensoren zum Erfassen der Dezentrierung des optischen Systems und/oder der Position und Orientierung des optischen Systems und/oder des mindestens einen im Gesichtsfeld angeordneten Objekts ausgestattet. Hierdurch werden entsprechend dem erfindungsgemäßen Verfahren die Lagedaten für die Justierung durch den Bildprozessor erfasst.

In einer Ausführungsform erfolgt das Einstellen der entsprechenden Verschiebung derart, dass durch die geometrische Entzerrung verarbeitete Bildinformationen entsprechend der Lagedaten verschoben werden.

In einer dazu alternativen Ausführungsform erfolgt das Einstellen der entsprechenden Verschiebung derart, dass entsprechend der Lagedaten verschobene Bildinformationen durch die geometrische Entzerrung verarbeitet werden. In einer bevorzugten Variante der beiden vorherigen Ausführungsformen, kann zwischen den beiden Ausführungsformen gewählt werden. Hierdurch kann ein Nutzer des optischen Systems eine Reihenfolge aus Verschiebung und Entzerrung wählen, die für das zu untersuchende Objekt oder den vorliegenden Sachverhalt besonders geeignet ist. In einer besonders bevorzugten Ausführungsvariante ist das optische System stereoskopisch, und es wird die Bildinformation entsprechend der vertikalen und/oder horizontalen Bildkoordinaten verschoben.

In einer weiteren Ausführungsform liegt ein stereoskopisches optisches System vor und es wird dem Bildprozessor eine Differenz der vertikalen Bildkoordinaten für das Justieren bereitgestellt. Hierbei kann unter der Annahme einer gleichstarken Dejustierung beider Halbbilder eine Abschätzung einer tatsächlichen Verschiebung bestimmt werden.

In einer weiteren Ausführungsform werden die Einstellung der geometrischen Entzerrung und/oder die Einstellung der Verschiebung als Justierparameter gespeichert. Hierdurch kann vorteilhaft zu einem späteren Zeitpunkt auf die entsprechenden Justierparameter zurückgegriffen werden. Dies erlaubt eine geringe Datenrate für die Durchführung des erfindungsgemäßen Verfahrens zur Justage, und daher auch eine kurze Verfahrensdauer. Besonders vorteilhaft ist die Variante dieser Ausführungsform, in der mindestens die Einstellung der geometrischen Entzerrung gespeichert wird, da hierdurch dieser Verfahrensschritt nur selten ausgeführt werden braucht. Hierbei wird der Umstand ausgenutzt, dass die geometrische Entzerrung nicht vor jedem Erfassen des zu untersuchenden Objekts durchgeführt werden muss, wenn entsprechende Justierparameter bereits gespeichert vorliegen und genutzt werden können. In einer Variante wird die Einstellung der geometrischen Entzerrung, also der statischen Justierschritte, nur einmalig nach der Herstellung des entsprechenden optischen Systems, durchgeführt. Hierbei werden die dazugehörigen Justierparameter gespeichert und vom Nutzer des optischen Systems werden lediglich die dynamischen Justierschritte ausgeführt.

In einer Ausführungsform für ein stereoskopisches optisches System werden das erste und zweite Halbbild des vorbestimmten Referenzobjekts durch das Sensorsystem erfasst, und das Justieren des Bildprozessors erfolgt durch einen Vergleich der auf dem ersten Halbbild abgebildeten mindestens zwei bekannten sichtbaren ReferenzobjektMerkmale mit den auf dem zweiten Halbbild abgebildeten mindestens zwei bekannten sichtbaren Referenzobjekt-Merkmalen. Hierdurch kann die optische Verzeichnung besonders genau bestimmt werden, wodurch eine sehr genaue geometrische Entzerrung eingestellt werden kann. In einer bevorzugten Variante werden mehr als zwei, bevorzugt mehr als acht, bekannte sichtbare Referenzobjekt-Merkmale auf dem ersten und zweiten Halbbild miteinander verglichen.

In einer weiteren Variante der vorherigen Ausführungsform weist das Verfahren neben dem Ermitteln der optischen Verzeichnung ein Ermitteln einer Vergrößerungsdifferenz zwischen dem ersten und zweiten Halbbild, oder einer relativen Verschiebung zwischen dem ersten und zweiten Halbbild, oder einer Rotation zwischen dem ersten und zweiten Halbbild, oder eine Kombination daraus auf. In einem bevorzugten Beispiel dieser Variante werden alle entsprechend ermittelten Justierparameter genutzt, um eine gleichmäßige Vergrößerung des ersten und zweiten Halbbilds, einen Ausgleich zur relativen Verschiebung zwischen dem ersten und zweiten Halbbild, oder einen Ausgleich zur Rotation zwischen dem ersten und zweiten Halbbild, oder eine Kombination daraus einzustellen. Hierdurch kann die Qualität der entsprechend dem Verfahren durchgeführten Justage weiter verbessert werden. Das Ermitteln der relativen Verschiebung kann dabei durch ein Bestimmen von vertikalen und horizontalen Bildkoordinaten der bekannten sichtbaren Referenzobjekt-Merkmale wie während des Betriebs des optischen Systems realisiert werden. Hierdurch wird vorteilhaft eine statische Vorkorrektur des ersten und zweiten Halbbildes bereitgestellt, so dass während des Betriebs des optischen Geräts nur kleine Verschiebungen des ersten und zweiten Halbbildes gegeneinander durch den Bildprozessor durchgeführt werden müssen. Hierdurch kann die Verfahrensdauer während des Betriebs zum Erfassen eines zu untersuchenden Objekts deutlich reduziert werden.

In einer alternativen Ausführungsform wird nur das erste Halbbild durch das Sensorsystem erfasst und für die Bestimmung der Positionsinformation der mindestens zwei sichtbaren Referenzobjekt-Merkmale genutzt. Hierdurch kann die Verfahrensdauer für die mittels Referenzobjekt durchgeführten statischen Verfahrensschritte reduziert werden. In einer Variante dieser Ausführungsform wird das erste Halbbild zur geometrischen Entzerrung beider Halbbilder verwendet.

In einer weiteren Ausführungsform wird der mindestens eine Referenzpunkt durch ein Bereitstellen eines auf das zu untersuchende Objekt gerichteten Lichtstrahls gebildet. Hierdurch ist das Verfahren für eine besonders große Vielfalt an zu untersuchenden Objekten anwendbar, da dass Objekt keine eigenen Untersuchungsobjekt-Merkmale aufzuweisen braucht, die als Referenzpunkte dienen. Weiterhin sind gemäß dieser Ausführungsform keine aufwendigen Anpassungsschritte beispielsweise des Sensorsystems an vorbestimmte sichtbaren Referenzpunkte notwendig, oder nur einmalig notwendig. Die Nutzung eines Lichtstrahls führt auch vorteilhaft dazu, dass keine Markierung als Untersuchungsobjekt-Merkmal an dem zu untersuchenden Objekt angebracht werden muss, wenn das Objekt keine geeigneten Untersuchungsobjektmerkmale aufweist. In einer Variante dieser Ausführungsform wird der Lichtstrahl in einem vorbestimmten Spektralbereich bereitgestellt, so dass das Sensorsystem ein entsprechendes Bild des Lichtstrahls empfangen kann und der Lichtstrahl nicht vom Nutzer des optischen Systems wahrgenommen wird. In einer anderen Variante dieser Ausführungsform wird der Lichtstrahl gepulst bereitgestellt, so dass der Lichtstrahl vom Nutzer des optischen Systems für einen Großteil einer Untersuchungsdauer nicht wahrgenommen wird. Hierdurch wird das Erfassen des zu untersuchenden Objekts durch das optische System besonders wenig oder gar nicht beeinträchtigt. In einer Variante bildet der Lichtstrahl eine strukturierte Beleuchtung des Objekts. Unter einer strukturierten Beleuchtung wird eine Beleuchtung mit einer vorbestimmten und nicht homogenen Intensitätsverteilung einer Intensität der Lichtstrahlen auf dem zu beleuchtenden Objekt oder in dem zu beleuchtenden Gesichtsfeld verstanden. Hierdurch kann eine Vielzahl von Referenzpunkten besonders einfach bereitgestellt werden.

Zur Lösung der vorgenannten Aufgabe wird gemäß einem zweiten Aspekt ein Justiersystem zur Verwendung in einem optischen System, insbesondere in einem stereoskopischen optischen System zum Erfassen eines zu untersuchenden Objekts vorgeschlagen. Das Justiersystem weist ein vorbestimmtes Referenzobjekt mit bekannten sichtbaren Referenzobjekt-Merkmalen, ein Sensorsystem, ein Datenauswertungs-Modul und einen Bildprozessor auf.

Das Sensorsystem ist ausgebildet, Bilddaten eines durch das optische System bereitgestellten Bildes oder, bei einem stereoskopischen System, eines ersten und zweiten Halbbildes eines in einem durch das optische System abzubildenden Gesichtsfeld angeordneten Objekts zu erfassen, wobei das erste und das zweite Halbbild auf einer Abbildungsfläche des Sensorsystems abgebildet werden. Das Sensorsystem kann insbesondere ein Kamerasystem sein.

Das Datenauswertungs-Modul ist mit dem Sensorsystem verbunden und ausgebildet, die erfassten Bilddaten zu empfangen und aus den Bilddaten eine Positionsinformation mindestens zweier sichtbarer Referenzobjekt-Merkmale des in dem Gesichtsfeld angeordneten vorbestimmten Referenzobjekts zu bestimmen. Weiterhin ist das Datenauswertungs-Modul ausgebildet, Lagedaten zu bestimmen, welche eine durch mindestens einen Referenzpunkt bestimmte Lageabweichung eines durch das optische System bereitgestellten Bildes des zu untersuchenden Objekts gegenüber erfassten Lagedaten früherer Zeitpunkte zur Ermittlung einer Beschleunigungsinformation des optischen Systems oder bei einem stereoskopischen optischen System einer gegenseitigen Lage eines ersten und zweiten Halbbildes des zu untersuchenden Objekts durch das Sensorsystem enthalten.

Der Bildprozessor ist mit dem Datenauswertungs-Modul verbunden und ausgebildet, aus der Positionsinformation der mindestens zwei sichtbaren Referenzobjekt-Merkmale des Referenzobjekts eine optische Verzeichnung des Bildes oder des ersten und/oder zweiten Halbbildes zu ermitteln und eine entsprechende geometrische Entzerrung von empfangenen Bilddaten einzustellen. Weiterhin ist der Bildprozessor ausgebildet, entsprechend der erfassten Lageabweichung eine entsprechende Verschiebung von empfangenen Bilddaten des Bildes oder des ersten und zweiten Halbbildes einzustellen.

Vorteilhaft erlaubt das erfindungsgemäße Justiersystem daher eine verbesserte Justierung und Verzeichnungskorrektur des Bildes oder der beiden stereoskopischen Halbbilder eines optischen Systems. Hierbei können das Datenauswertungs-Modul und der Bildprozessor sowohl für die statischen Justierschritte, die mit dem Referenzobjekt durchgeführt werden, als auch für die dynamischen Justierschritte, während des Betriebs des optischen Geräts, genutzt werden.

Da das erfindungsgemäße Justiersystem kaum Anforderungen an das stereoskopische optische System stellt, kann es in einer Vielzahl von Anwendungsbereichen, insbesondere stereoskopischen Anwendungsbereichen genutzt werden. Dabei benötigt das Justiersystem eine so geringe Anzahl an Nutzereingaben, wie beispielsweise eine Aktivierung oder Deaktivierung, dass es von Nutzern des optischen Systems, also beispielsweise medizinischem Fachpersonal, auch ohne Spezialkenntnisse auf dem Gebiet der Kamerajustierung oder -kalibrierung schnell, sicher und unkompliziert genutzt werden kann.

Nachfolgend werden bevorzugte Ausführungsformen des erfindungsgemäßen Justiersystems gemäß dem zweiten Aspekt der Erfindung genannt.

In einer bevorzugten Ausführungsform ist das Datenauswertungsmodul zur Nutzung in einem stereoskopischen optischen Systems ausgebildet, in dem ersten Halbbild des zu untersuchenden Objekts eine erste vertikale und/oder horizontale Bildkoordinate eines Untersuchungsobjekt-Merkmals des zu untersuchenden Objekts und in dem zweiten Halbbild des zu untersuchenden Objekts eine zweite endsprechend vertikale und/oder horizontale Bildkoordinate des Untersuchungsobjekt-Merkmals zu bestimmen. Hierbei wird eine etwaige horizontale Bildkoordinate bezüglich einer horizontalen Bildachse bestimmt, die in einer Bildebene des stereoskopischen optischen Systems liegt und beide optische Achsen des stereoskopischen optischen Systems schneidet. Eine etwaige vertikale Bildkoordinate wird weiterhin bezüglich einer vertikalen Bildachse bestimmt, die in der Bildebene des optischen Systems und senkrecht zur horizontalen Bildachse liegt.

Die jeweiligen vertikalen oder horizontalen Bildkoordinaten können im Rahmen der Erfindung entlang der vertikalen oder horizontalen Bildachse bestimmt werden, aber auch entlang einer Richtung, die im Wesentlichen entlang der vertikalen oder horizontalen Bildachse orientiert ist, jedoch nicht mit dieser übereinstimmt.

Weiterhin können die vertikalen oder horizontalen Bildkoordinaten direkt aus einer Abbildung des zu untersuchenden Objekts oder aus durch Verarbeitung der Abbildung gewonnenen Untersuchungsobjekt-Daten als virtuelle Bildkoordinaten ermittelt werden.

In einer bevorzugten Ausführungsform weist das Justiersystem weiterhin ein SpeicherModul auf, das mit dem Bildprozessor verbunden ist und ausgebildet ist, die Einstellung der geometrischen Entzerrung und/oder die Einstellung der Verschiebung als Justierparameter zu speichern. Hierdurch kann zu einem späteren Zeitpunkt während einer Nutzung des Justiersystems auf die Justierparameter zurückgegriffen werden. Als Justierparameter wird hierbei auch ein Satz von Justierparametern betrachtet, die eine konkrete Einstellung betreffen. Vorzugsweise kann die Einstellung der geometrischen Entzerrung gespeichert werden, wodurch die Bestimmung der Positionsinformation nicht für jedes Erfassen eines zu untersuchenden Objekts durch das optische System notwendig ist. Daher führt ein Speicher-Modul zu einer geringen Justagedauer des Justiersystems, was insbesondere für sich bewegende zu untersuchende Objekte vorteilhaft ist. In einer Variante dieser Ausführungsform wird das Speichermodul zusätzlich oder alternativ verwendet, um auf die erfassten Lagedaten früherer Zeitpunkte zuzugreifen und dadurch eine Beschleunigungsinformation zu bestimmen. Hierbei wird eine Position des mindestens einen Referenzpunkts zu mindestens zwei verschiedenen Zeitpunkten bestimmt. Dies ist besonders vorteilhaft für das Justieren des Bildprozessors bei Vorliegen einer regelmäßigen Bewegung des optischen Systems und/oder des zu untersuchenden Objekts, wie beispielsweise bei einem Wackeln des optischen Systems.

In einer weiteren bevorzugten Ausführungsform ist das vorbestimmte Referenzobjekt eine Messtafel, die vorbestimmte geometrische Formen in vorbestimmter Weise darstellt. In anderen Ausführungsformen ist das Referenzobjekt eine bedruckte Folie, ein strukturierter Hintergrund oder ein vorbestimmt angeordneter geometrischer Körper. Die vorbestimmten geometrischen Formen können vorteilhaft Punkte, Rechtecke oder andere Polygonzüge sein. In einer besonders vorteilhaften Variante, ist das DatenauswertungsModul dazu ausgebildet, eine Durchführung des statischen Justierschrittes durch Bestimmung der Positionsinformation dann auszulösen, wenn mindestens ein vordefiniertes Referenzobjekt-Merkmal von dem Sensorsystem detektiert wird. Hierdurch kann automatisiert die Positionsinformation genau dann bestimmt werden, wenn das Referenzobjekt in der abzubildenden Gesichtsfeld angeordnet ist, was eine diesbezügliche Nutzereingabe erspart.

In einer weiteren Ausführungsform weist das Justiersystem weiterhin eine Beleuchtungseinrichtung auf, die ausgebildet ist, einen auf das zu untersuchende Objekt gerichteten Lichtstrahl bereitzustellen. Hierdurch kann für die zu untersuchenden Objekte ein sichtbares Untersuchungsobjekt-Merkmal einfach und in reproduzierbarer Weise bereitgestellt werden. In einer Variante dieser Ausführungsform werden mindestens 2 auf das zu untersuchende Objekt gerichtete Lichtstrahlen bereitgestellt. In einer weiteren Variante ist die Beleuchtungseinrichtung ausgebildet, den Lichtstrahl gepulst und/oder in einem für Nutzer des optischen Systems nicht wahrnehmbaren Spektralbereich bereitzustellen. Hierdurch kann sichergestellt werden, dass ein Erfassen des zu untersuchenden Objekts durch das erfindungsgemäße Justiersystem nicht beeinträchtigt wird. In einer Variante bildet der Lichtstrahl eine strukturierte Beleuchtung des Objekts.

In einer weiteren Ausführungsform ist ein Justage-Modul des Bildprozessors dazu ausgebildet, die geometrische Entzerrung sowie die Verschiebung von empfangen Bilddaten einzustellen. Das Justage-Modul ist in einer Variante dieser Ausführungsform in einem Gehäuse des Bildprozessors angeordnet. In einer anderen Variante ist das Justage-Modul räumlich getrennt von einem restlichen Teil des Bildprozessors angeordnet, aber mit diesem elektrisch verbunden.

Zur Lösung der vorgenannten Aufgabe wird gemäß einem dritten Aspekt eine Verwendung einer Ausführungsform des Justiersystems gemäß dem zweiten Aspekt der Erfindung in einem Regelkreis zur dynamischen Kompensation von Dejustierungen eines optischen Systems, insbesondere eines stereoskopischen optischen Systems, insbesondere eines Stereoendoskops, vorgeschlagen.

Die Verwendung des erfindungsgemäßen Justiersystem ist hierbei besonders vorteilhaft, da es wenig Nutzereingaben benötigt und daher weitgehend automatisch genutzt werden kann. Beispielsweise kann nach einer Aktivierung des Justiersystems durch einen Nutzer der Regelkreis automatisiert Dejustierungen des optischen Systems kompensieren.

In einer Ausführungsform ist die dynamische Kompensation von Dejustierungen derart ausgebildet, dass die ermittelten Einstellungen der geometrischen Entzerrung und/oder der entsprechenden Verschiebung durch den Bildprozessor derart verwendet werden, dass elektronische Bildmasken zur Begrenzung eines auszugebenden Bildfeldes entsprechend der Einstellungen angeordnet werden.

Weiterhin wird zur Lösung der vorgenannten Aufgabe gemäß einem vierten Aspekt ein medizinisches Gerät, insbesondere ein medizinisches stereoskopisches Gerät, insbesondere ein Stereoendoskop, vorgeschlagen, das eine Ausführungsform des Justiersystem gemäß dem zweiten Aspekt der Erfindung aufweist.

Ein medizinisches stereoskopisches Gerät gemäß dem vierten Aspekt der Erfindung ist besonders vorteilhaft, da es dem Nutzer, also dem medizinischen Fachpersonal erlaubt, eine Justage mit dem Justiersystem ohne Spezialkenntnisse auf dem Gebiet der Kamerajustierung oder-kalibrierung schnell, sicher und unkompliziert auszuführen.

Gemäß einem fünften Aspekt der Erfindung wird zur Lösung der vorgenannten Aufgabe ein Computer-Programm vorgeschlagen, das zum Steuern einer Ausführungsform des Verfahrens gemäß dem ersten Aspekt der Erfindung durch einen Computer geeignet ist.

Das Computer-Programm wird bevorzugt auf einem Prozessor ausgeführt, der einen Teil des Bildprozessors bildet. Bevorzugt ist der Prozessor dabei ein Teil eines Stereoendoskopie-Systems.

Die Erfindung soll nun anhand von in den Figuren schematisch dargestellten Ausführungsbeispielen näher erläutert werden. Von den Figuren zeigen:
- Fig. 1: ein erstes Ausführungsbeispiel eines Justiersystems gemäß dem zweiten Aspekt der Erfindung;
- Fig. 2: ein zweites Ausführungsbeispiel des Kalibriersystems gemäß dem zweiten Aspekt der Erfindung;
- Fig. 3: ein Ausführungsbeispiel eines Verfahrens gemäß dem ersten Aspekt der Erfindung;
- Fig. 4: eine Veranschaulichung eines Bestimmens von horizontalen und vertikalen Bildkoordinaten gemäß dem Ausführungsbeispiels des Verfahrens gemäß dem ersten Aspekt der Erfindung;
- Figs. 5a, b: eine Veranschaulichung eines Einstellens einer geometrischen Entzerrung und einer Verschiebung von Halbbildern gemäß einer ersten Variante des Ausführungsbeispiels des Verfahrens gemäß dem ersten Aspekt der Erfindung; und
- Figs. 6a, b: eine Veranschaulichung eines Einstellens der geometrischen Entzerrung und der Verschiebung von Halbbildern gemäß einer zweiten Variante des Ausführungsbeispiels des Verfahrens gemäß dem ersten Aspekt der Erfindung.

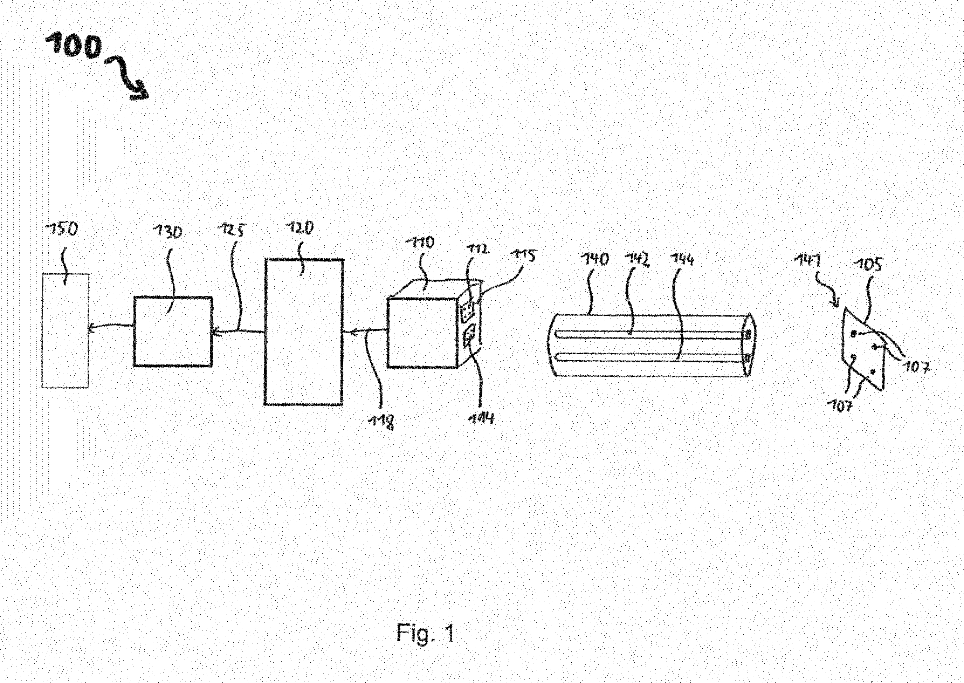

Fig. 1 zeigt ein erstes Ausführungsbeispiel eines Justiersystems 100.

Das Justiersystem 100 umfasst ein vorbestimmtes Referenzobjekt 105, ein Sensorsystem 110, ein Datenauswertungs-Modul 120 und einen Bildprozessor 130.

Das Referenzobjekt 105 hat bekannte sichtbare Referenzobjekt-Merkmale 107. Das Referenzobjekt 105 ist dabei so angeordnet, dass die Referenzobjekt-Merkmale 107, die im gezeigten Ausführungsbeispiel als Punkte auf einer Messtafel ausgebildet sind, von einem stereoskopischen optischen System 140 abgebildet werden können.

Das stereoskopische optische System 140 ist im dargestellten Ausführungsbeispiel ein Stereoendoskop mit einem ersten 142 und zweiten 144 optischen Kanal, durch die ein erstes 112 und zweites Halbbild 114 des Referenzobjekts 105 auf einer Abbildungsfläche 115 des Sensorsystems 110 abgebildet werden kann. In nicht dargestellten Ausführungsbeispielen ist das optische System monoskopisch ausgebildet.

Das Sensorsystem 110 ist dabei ausgebildet, Bilddaten 118 des auf der Abbildungsfläche 115 abgebildeten ersten 112 und zweiten Halbbildes 114 zu erfassen. In dem dargestellten Ausführungsbeispiel ist das Sensorsystem 110 ein CCD-Kamerasystem. In anderen Ausführungsbeispielen werden andere Kamerasysteme, wie beispielsweise CMOS-Kamerasysteme genutzt. Objekte werden genau dann auf der Abbildungsfläche 115 abgebildet, wenn sie in einem Gesichtsfeld 141 des optischen Systems 140 angeordnet sind.

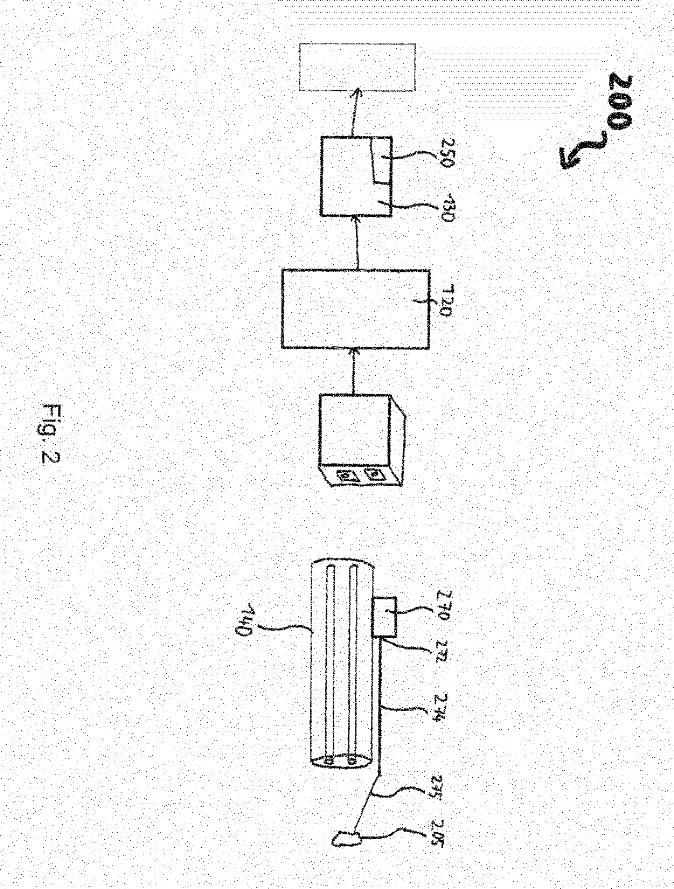

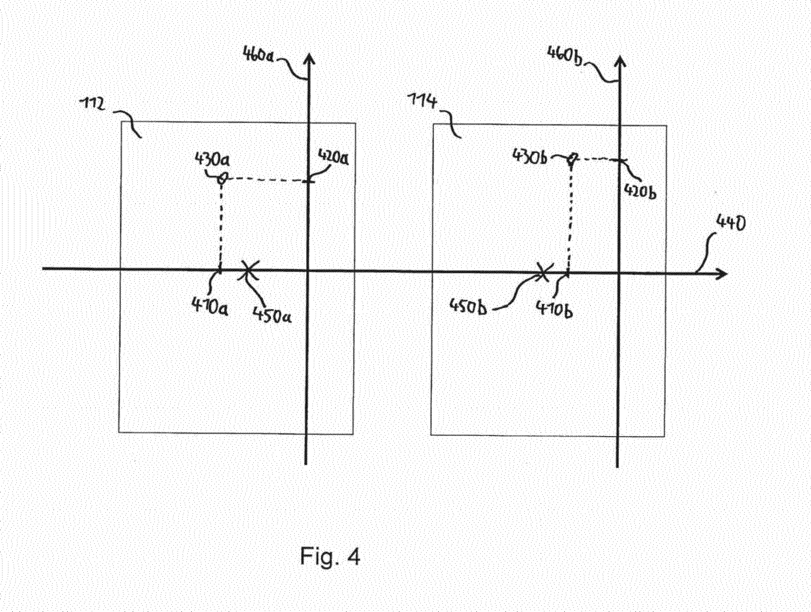

Das Datenauswertungs-Modul 120 ist mit dem Sensorsystem 110 elektrisch verbunden und ausgebildet, die erfassten Bilddaten 118 zu empfangen. Für den in Fig. 1 dargestellten Fall, dass das Referenzobjekt 105 in dem Gesichtsfeld 141 angeordnet ist, ist das Datenauswertungs-Modul 120 ausgebildet, aus den Bilddaten 118 eine Positionsinformation 125 mindestens zweier sichtbarer Referenzobjekt-Merkmale 107 des in dem Gesichtsfeld 141 angeordneten vorbestimmten Referenzobjekts 105 zu bestimmen. Hierfür werden vom Datenauswertungs-Modul 120 Bilddaten 118 des ersten und zweiten Halbbildes 112, 114 genutzt. Diese beschriebene statische Erfassung von ReferenzobjektMerkmalen 107 stellt typischerweise erste, statische Justierschritte einer mit dem Justiersystem durchzuführenden Justage dar. Im zweiten, in Fig. 2 dargestellten, dynamischen Justierschritten werden Bilddaten 118 des zu untersuchenden Objekts durch das Datenauswertungs-Modul 120 empfangen. Das Datenauswertungs-Modul 120 ist dabei ausgebildet, in dem ersten Halbbild 112 des zu untersuchenden Objekts eine erste vertikale und/oder horizontale Bildkoordinate eines Untersuchungsobjekt-Merkmals des zu untersuchenden Objekts und in dem zweiten Halbbild 114 des zu untersuchenden Objekts eine zweite endsprechend vertikale und horizontale Bildkoordinate des Untersuchungsobjekt-Merkmals zu bestimmen, was im Rahmen von Fig. 4 näher erläutert ist. Die Bildkoordinaten beschreiben daher Lagedaten, die eine durch mindestens einen Referenzpunkt bestimmte Lageabweichung des ersten und zweiten Halbbildes enthalten. Hierbei wird die horizontale Bildkoordinate bezüglich einer horizontalen Bildachse bestimmt, die auf der Abbildungsfläche 115 des stereoskopischen optischen Systems 140 liegt und beide optische Achsen des ersten 142 und zweiten Kanals 144 des stereoskopischen optischen Systems 140 schneidet. Die vertikale Bildkoordinate wird bezüglich einer vertikalen Bildachse bestimmt, die auf der Abbildungsfläche 115 des optischen Systems 140 und senkrecht zur horizontalen Bildachse liegt.

Der Bildprozessor 130 ist mit dem Datenauswertungs-Modul 120 elektrisch verbunden. Dabei ist er ausgebildet, aus der Positionsinformation 125 der mindestens zwei sichtbaren Referenzobjekt-Merkmale 107 des Referenzobjekts 105 eine optische Verzeichnung des ersten und zweiten Halbbildes 112, 114 zu ermitteln und eine entsprechende geometrische Entzerrung zur Kompensation der optischen Verzeichnung von empfangenen Bilddaten einzustellen. Zusätzlich zur optischen Verzeichnung wird in dem dargestellten Ausführungsbeispiel eine Vergrößerungsdifferenz zwischen dem ersten und zweiten Halbbild 112, 114 aus der Positionsinformation 125 ermittelt und zum Einstellen der geometrischen Entzerrung genutzt.

Im Rahmen der zweiten, dynamischen Justierschritte ist der Bildprozessor 130 ausgebildet, entsprechend der Lagedaten, also entsprechend einer Differenz aus der ersten und zweiten vertikalen Bildkoordinate und aus der ersten und zweiten horizontalen Bildkoordinate eine entsprechende Verschiebung von empfangenen Bilddaten des ersten und zweiten Halbbildes gegeneinander einzustellen.

Das erfindungsgemäße Justiersystem 100 ist daher ausgebildet, im Rahmen der ersten, statischen Justierschritte über das Erfassen von Bilddaten 118 des Referenzobjektes 105 die geometrische Entzerrung von zukünftig empfangenen Bilddaten einzustellen. Weiterhin ist das Justiersystem 100 ausgebildet, durch darauffolgende dynamische Justierschritte über das Erfassen eines zu untersuchenden Objekts die entsprechende Verschiebung von empfangenen Bilddaten des ersten und zweiten Halbbildes gegeneinander einzustellen. Hierbei ist das Justiergerät in eine Bildverarbeitung des optischen Systems 140 integriert, so dass der Bildprozessor mit einer als Bildschirm ausgebildeten Ausgabeeinheit 150 verbunden ist, die eine optische Ausgabe basierend auf den empfangenen Bilddaten bereitstellt. Das optische System 140 und die Ausgabeeinheit 150 sind in dem dargestellten Ausführungsbeispiel nicht Teil des Justiersystems 100.

In der in Fig. 1 dargestellten Ausführungsform kann das Justiergerät daher zur dynamischen Kompensation von Dejustierungen des stereoskopischen optischen Systems 100 verwendet werden.

Die dargestellte Verwendung des Justiersystems 100 für ein starres Stereoendoskop stellt ein bevorzugtes Einsatzgebiet der Erfindung dar. Weiterhin kann das Justiersystem allerdings auch für andere monoskopische und stereoskopische optische Systeme zur Justage verwendet werden.

Fig. 2 zeigt ein zweites Ausführungsbeispiel des Justiersystems 200.

Im Unterschied zu dem in Fig. 1 gezeigten Justiersystem 100, weist das Justiersystem 200 ein Speicher-Modul 250 auf, das mit dem Bildprozessor 130 elektrisch verbunden ist. In nicht gezeigten Ausführungsbeispielen bildet das Speicher-Modul einen Teil des Bildprozessors.

Das Speicher-Modul 250 ist ausgebildet, die Einstellung der geometrischen Entzerrung und die Einstellung der Verschiebung als Justierparameter zu speichern. In anderen Ausführungsbeispielen wird durch das Speicher-Modul nur die geometrische Entzerrung als Justierparameter gespeichert, wohingegen die Verschiebung immer aktuell eingestellt und nicht gespeichert wird.

Ein weiterer Unterschied zu dem Justiersystem 100 aus Fig. 1, bildet die Beleuchtungseinrichtung 270, die einen Teil des Justiersystems 200 darstellt. Die Beleuchtungseinrichtung 270 ist ausgebildet, einen auf das zu untersuchende Objekt 205 gerichteten Lichtstrahl 275 bereitzustellen. Vorliegend wird der Lichtstrahl durch eine LED 272 und einen entsprechenden Lichtleiter 274 bereitgestellt. Hierdurch wird der Referenzpunkt, welcher von dem Datenauswertungs-Modul 120 zu erfassen ist, besonders einfach und vorteilhaft bereitgestellt. In einem nicht dargestellten Ausführungsbeispiel stellt die Beleuchtungseinrichtung regelmäßig kurze Lichtimpulse bereit, so dass der Nutzer des optischen Systems 140 nicht dauerhaft beim Betrachten des zu untersuchenden Objekts durch ein Bild des Lichtstrahls gestört wird. Während der Dauer des Lichtimpulses kann hierbei in einer Variante des nicht dargestellten Ausführungsbeispiels die Bildwiedergabe des optischen Systems 140 unterbrochen werden.

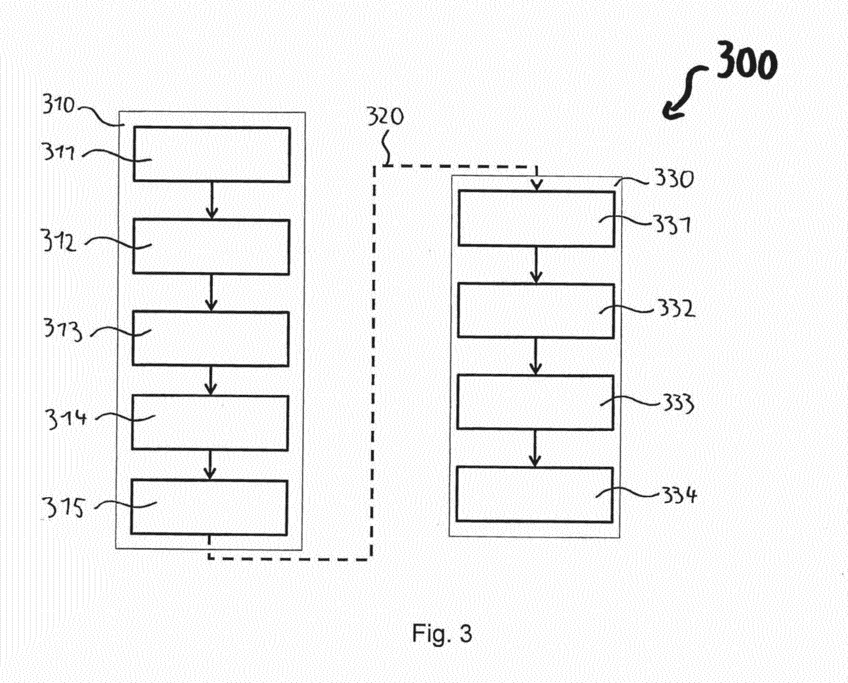

Fig. 3 zeigt ein Ausführungsbeispiel eines Verfahrens 300 gemäß dem ersten Aspekt der Erfindung. Dieses Verfahren 300 zum Justieren eines stereoskopischen optischen Systems weist 5 Schritte 311, 312, 313, 314, 315 auf, die die ersten, statischen Justierschritte 310 bilden, und 4 Schritte 331, 332, 333, 334, die die zweiten, dynamischen Justierschritte 330 bilden. Ein Übergang 320 zwischen dem letzten der ersten, statischen 310 und dem ersten der zweiten, dynamischen 330 Justierschritte kann sich über einen langen Zeitraum erstrecken, aber auch sehr kurz sein. Dies hängt davon ab, wie lang der Zeitraum ist, den ein Nutzer eines das Verfahren teilweise ausführenden Systems benötigt, um nach einem Erfassen eines Referenzobjekts ein zu untersuchendes Objekt durch das stereoskopische optisches System zu betrachten.

Die ersten, statischen Justierschritte 310 umfassen die im Folgenden vorgestellten Schritte.

In einem ersten Schritt 311 wird ein vorbestimmtes Referenzobjekt mit bekannten sichtbaren Referenzobjekt-Merkmalen im durch das optische System abzubildenden Gesichtsfeld angeordnet.

In einem weiteren Schritt 312 wird ein durch das optische System bereitgestelltes erstes und/oder zweites Halbbild des vorbestimmten Referenzobjekts durch ein Sensorsystem erfasst.

In einem nächsten Schritt 313 wird eine Positionsinformation mindestens zweier in dem ersten und/oder zweiten Halbbild abgebildeter sichtbarer Referenzobjekt-Merkmale des vorbestimmten Referenzobjekts bestimmt.

Weiterhin wird in einem folgenden Schritt 314 eine optische Verzeichnung des ersten und/oder zweiten Halbbildes aus der Positionsinformation der mindestens zwei sichtbaren Referenzobjekt-Merkmale ermittelt.

In einem letzten Schritt 315 der ersten, statischen Justierschritte 310 wird ein mit dem Sensorsystem zumindest indirekt verbundener Bildprozessors, durch ein Einstellen einer geometrischen Entzerrung entsprechend der ermittelten optischen Verzeichnung justiert.

Während eines Betriebs des optischen Systems zum Erfassen eines zu untersuchenden Objekts weist das Verfahren im Rahmen der zweiten, dynamischen Justierschritte 330 die im Folgenden erläuterten Schritte auf.

Zuerst weisen die dynamischen Justierschritte 330 in einem ersten Schritt 331 ein Anordnen des zu untersuchenden Objekts in dem durch das optische System abzubildenden Gesichtsfeld auf.

In einem weiteren Schritt 332 werden Lagedaten erfasst, welche eine durch mindestens einen Referenzpunkt bestimmte Lageabweichung eines ersten und zweiten Halbbildes des zu untersuchenden Objekts durch das Sensorsystem enthalten.

In einem darauffolgenden Schritt 333 wird eine erste vertikale und/oder horizontale Bildkoordinate des Referenzpunktes in dem ersten Halbbild und eine zweite entsprechend vertikale und/oder horizontale Bildkoordinate des Referenzpunktes in dem zweiten Halbbild bestimmt. Hierbei wird eine etwaige horizontale Bildkoordinate bezüglich einer horizontalen Bildachse bestimmt, die innerhalb einer Bildebene des stereoskopischen optischen Systems liegt und beide optische Achsen des stereoskopischen optischen Systems schneidet. Eine etwaige vertikale Bildkoordinate wird wiederum bezüglich einer vertikalen Bildachse bestimmt, die innerhalb der Bildebene des optischen Systems und senkrecht zur horizontalen Bildachse liegt.

Ein letzter Schritt 334 des Verfahrens 300 besteht aus einem Justieren des Bildprozessors entsprechend der erfassten Lageabweichung, also entsprechend einer Differenz aus der ersten und zweiten vertikalen Bildkoordinate und/oder aus der ersten und zweiten horizontalen Bildkoordinate, durch Einstellen einer entsprechenden Verschiebung des ersten und zweiten Halbbildes gegeneinander durch den Bildprozessor.

Das Verfahren kann in einigen Varianten dieses Ausführungsbeispiels auch derart ausgeführt werden, dass die statischen Justierschritte 310 deutlich seltener ausgeführt werden als die dynamischen Justierschritte 330. Hierbei werden die durch die statischen Justierschritte 310 gewonnen Einstellungen als Justierparameter gespeichert und nach den dynamischen Justierschritten 330 jeweils zum Justieren genutzt. Dadurch kann vorteilhaft die Dauer des gesamten Justiervorgangs verringert werden. Weiterhin kann eine Datenübertragungsrate zum Bildprozessor gering gehalten werden, was sich vorteilhaft auf die technischen Anforderungen an das entsprechende Justiersystem und an das optische System auswirkt.

Weiterhin kann das Verfahren auch mit einem monoskopischen optischen System ausgeführt werden, Hierbei wird die Lageabweichung nicht durch einen Vergleich zwischen dem ersten und zweiten Halbbild bestimmt, sondern durch mindestens einen Referenzpunkt eines durch das optischen System bereitgestellten Bildes des zu untersuchenden Objekts.

Fig. 4 zeigt eine Veranschaulichung eines Bestimmens von horizontalen und vertikalen Bildkoordinaten 410a, 410b, 420a, 420b gemäß dem Ausführungsbeispiel des Verfahrens 300.

Hierbei ist eine Draufsicht auf die Abbildungsfläche 115 des Sensorsystem 110 gezeigt, wobei das erste Halbbild 112 und das zweite Halbbild 114 getrennt voneinander abgebildet werden. Dabei wird das Untersuchungsobjekt-Merkmal 430a, 430b im ersten 112 und zweiten Halbbild 114 des stereoskopischen optischen Systems abgebildet.

Die horizontale Bildkoordinaten 410a, 410b des Untersuchungsobjekt-Merkmals 430a, 430b in dem ersten und zweiten Halbbild 112, 114 werden bezüglich einer horizontalen Bildachse 440 bestimmt. Diese horizontale Bildachse 440 liegt innerhalb der durch die Abbildungsfläche 115 gebildeten Bildebene des stereoskopischen optischen Systems und sie schneidet beide optische Achsen des stereoskopischen optischen Systems. Die Schnittpunkte 450a, 450b sind als Kreuze dargestellt.

Die vertikalen Bildkoordinaten 420a, 420b des Untersuchungsobjekt-Merkmals 430a, 430b werden bezüglich einer vertikalen Bildachse 460a, 460b bestimmt. Die vertikale Bildachse 460a, 460b liegt innerhalb der durch die Abbildungsfläche 115 gebildeten Bildebene des stereoskopischen optischen Systems und ist senkrecht zur horizontalen Bildachse 440. Da der Schnittpunkt der vertikalen Bildachse 460a, 460b mit der horizontalen Bildachse 440 unbedeutend ist, ist in Fig. 4 die vertikale Bildachse 460a, 460b sowohl für das erste Halbbild 112 als auch für das zweite Halbbild 114 aus Gründen der Übersichtlichkeit dargestellt.

In einem nicht dargestellten Ausführungsbeispiel ist die horizontale Bildachse nicht als Verbindung zwischen den Schnittpunkten der optischen Achsen des optischen Systems und der Abbildungsfläche ausgebildet, sondern bezüglich dieser Schnittpunkte in vorbestimmter Weise auf der Abbildungsfläche orientiert.

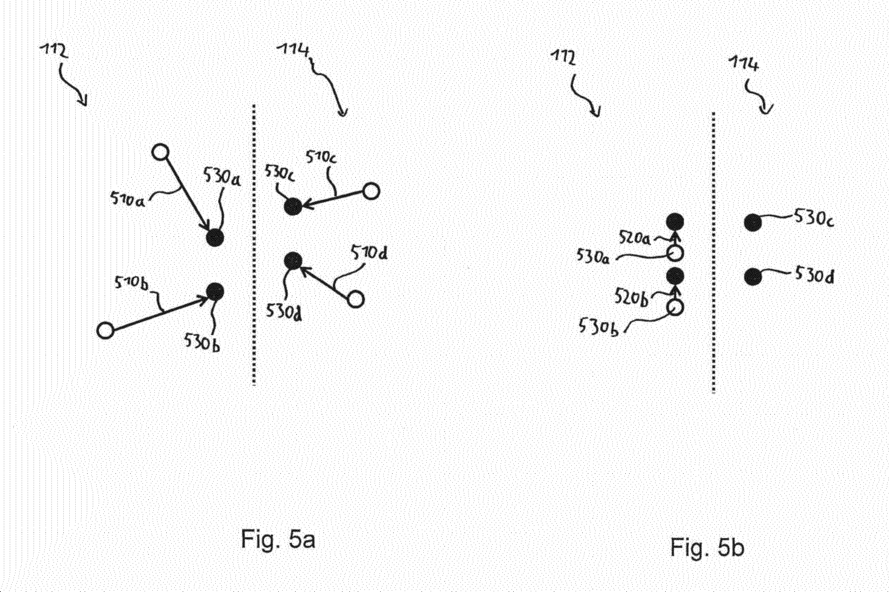

Figs. 5a, b zeigen eine Veranschaulichung eines Einstellens einer geometrischen Entzerrung 510a, 510b, 510c, 510d und einer Verschiebung 520a, 520b von Halbbildern 112, 114 gemäß einer ersten Variante des Ausführungsbeispiels des Verfahrens.

In der dargestellten Variante des Verfahrens 300 erfolgt das Einstellen der entsprechenden Verschiebung 520a, 520b derart, dass durch die geometrische Entzerrung 510a, 510b, 510c, 510d verarbeitete Bildinformationen 530a, 530b, 530c, 530d des ersten 112 und/oder zweiten Halbbildes 114 entsprechend der vertikalen und/oder horizontalen Bildkoordinaten verschoben werden.

Fig. 5a illustriert hierbei den Vorgang der geometrischen Entzerrung 510a, 510b, 510c, 510d, der nur innerhalb des Bildprozessors abläuft und daher in der vorliegenden Variante für den Anwender unsichtbar ist. Fig. 5b illustriert das darauffolgende Einstellen des ersten Halbbildes 112 durch ein Verschieben 520a, 520b des ersten Halbbildes 112 relativ zu dem zweiten Halbbild 114. Die so justierten Halbbilder können dem Anwender auf einem Stereobildschirm angezeigt werden. Der Anwender kann dann ein justiertes und unverzerrtes Stereobild sehen.

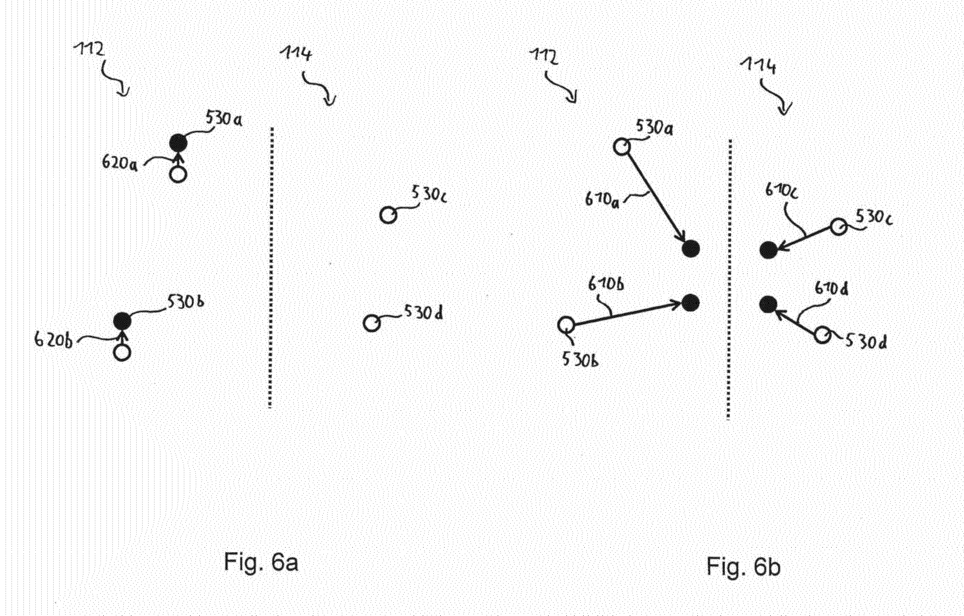

Figs. 6a, b zeigen eine Veranschaulichung eines Einstellens der geometrischen Entzerrung 610a, 610b, 610c, 610d und der Verschiebung 620a, 620b von Halbbildern 112, 114 gemäß einer zweiten Variante des Ausführungsbeispiels des Verfahrens.

In der dargestellten Variante des Verfahrens 300 erfolgt das Einstellen der entsprechenden Verschiebung 620a, 620b derart, dass entsprechend der vertikalen und/oder horizontalen Bildkoordinaten verschobene Bildinformationen 530a, 530b, 530c, 530d des ersten und/oder zweiten Halbbildes 112, 114 durch die geometrische Entzerrung 610a, 610b, 610c, 610d verarbeitet werden.

Fig. 6a illustriert hierbei den Vorgang des Verschieben 620a, 620b des ersten Halbbildes 112 relativ zu dem zweiten Halbbild 114. Fig. 6b illustriert das darauffolgende Einstellen der geometrischen Entzerrung 610a, 610b, 610c, 610d. Beide Einstellungen werden innerhalb des Bildprozessors ausgeführt und laufen daher intern ab. Die Figuren 5a, 5b, 6a und 6b dienen daher lediglich der Veranschaulichung von möglichen Varianten des Verfahrens 300. Die so justierten Halbbilder können hierbei dem Anwender auf einem Stereobildschirm angezeigt werden. Der Anwender sieht dann wiederum ein justiertes und unverzerrtes Stereobild.

Insbesondere werden in nicht dargestellten Varianten Bildverarbeitungsschritte durch den Bildprozessor ausgeführt, wobei zusätzlich die in Fig. 3 dargestellten Justierschritte parallel und/oder versetzt zu den Bildverarbeitungsschritten während eines Betriebs des optischen Systems ausgeführt werden.

### Bezuqszeichenliste:

- 100, 200: Justiersystem
- 105: Referenzobjekt
- 107: Referenzobjekt-Merkmale
- 110: Sensorsystem
- 112: erstes Halbbild
- 114: zweites Halbbild
- 115: Abbildungsfläche
- 118: Bilddaten
- 120: Datenauswertungs-Modul
- 125: Positionsinformation
- 130: Bildprozessor
- 140: optisches System
- 141: Gesichtsfeld
- 142: erster optischer Kanal
- 144: zweiter optischer Kanal
- 150: Ausgabeeinheit
- 205: zu untersuchendes Objekt
- 250: Speicher-Modul
- 270: Beleuchtungseinrichtung
- 272: LED
- 274: Lichtleiter
- 275: Lichtstrahl
- 300: Verfahren
- 310: statische Justierschritte
- 311, 312, 313 314, 315: jeweilige Schritte der statischen Justierschritte
- 320: Übergang des Verfahrens
- 330: dynamische Justierschritte
- 331, 332, 333 334: jeweilige Schritte der dynamischen Justierschritte
- 410a, 410b: horizontale Bildkoordinaten
- 420a, 420b: vertikale Bildkoordinaten
- 430a, 430b: Untersuchungsobjekt-Merkmal
- 440: horizontale Bildachse
- 450a, 450b: Schnittpunkte
- 460a, 460b: vertikale Bildachse
- 510a, 510b, 510c 510d: geometrische Entzerrung der ersten Variante
- 520a, 520b: Verschiebung der ersten Variante
- 530a, 530b, 530c 530d: verarbeitete Bildinformationen
- 610a, 610b, 610c 610d: geometrische Entzerrung der zweiten Variante
- 620a, 620b: Verschiebung der zweiten Variante

## Patentansprüche

1. Verfahren (300) zum Justieren eines optischen Systems (140), insbesondere eines stereoskopischen optischen Systems (140), aufweisend:
- Anordnen eines vorbestimmten Referenzobjekts (105) mit bekannten sichtbaren Referenzobjekt-Merkmalen (107) in einem durch das optische System (140) erfassten Gesichtsfeld (141);
- Erfassen eines durch das optische System (140) bereitgestellten Bildes des vorbestimmten Referenzobjekts (105) oder bei einem stereoskopischen optischen System (140) eines ersten und/oder zweiten Halbbildes (112, 114) des vorbestimmten Referenzobjekts (105) durch ein Sensorsystem (110), wobei das erste (112) und das zweite Halbbild (114) auf einer Abbildungsfläche (115) des Sensorsystems (110) abgebildet werden;
- Bestimmen einer Positionsinformation (125) mindestens zweier in dem Bild oder in dem ersten und/oder zweiten Halbbild (112, 114) abgebildeter sichtbarer Referenzobjekt-Merkmale (107) des vorbestimmten Referenzobjekts (105);
- Ermitteln einer optischen Verzeichnung des Bildes oder des ersten und/oder zweiten Halbbildes (112, 114) aus der Positionsinformation (125) der mindestens zwei sichtbaren Referenzobjekt-Merkmale (107);
- Justieren eines mit dem Sensorsystem (110) zumindest indirekt verbundenen Bildprozessors (130), durch Einstellen einer geometrischen Entzerrung entsprechend der ermittelten optischen Verzeichnung;
wobei das Verfahren (300) weiterhin während eines Betriebs des optischen Systems (140) zum Erfassen eines zu untersuchenden Objekts (205) die folgenden Schritte aufweist
- Anordnen des zu untersuchenden Objekts (205) im durch das optische System (140) erfassten Gesichtsfeld (141);
- Erfassen von Lagedaten, welche eine durch mindestens einen Referenzpunkt bestimmte Lageabweichung eines durch das optische System (140) bereitgestellten Bildes des zu untersuchenden Objekts (205) gegenüber erfassten Lagedaten früherer Zeitpunkte zur Ermittlung einer Beschleunigungsinformation des optischen Systems oder bei einem stereoskopischen optischen System (140) einer gegenseitigen Lage eines ersten und zweiten Halbbildes (112, 114) des zu untersuchenden Objekts (205) enthalten; und
- Justieren des Bildprozessors (130) entsprechend der erfassten Lageabweichung durch Einstellen einer entsprechenden Verschiebung des Bildes oder des ersten und/oder zweiten Halbbildes (112, 114) durch den Bildprozessor (130).

2. Verfahren (300) gemäß Anspruch 1, bei dem das optische System (140) stereoskopisch ist, bei dem das zu untersuchende Objekt (205) mindestens ein sichtbares Untersuchungsobjekt-Merkmal (430a, 430b) im durch das optische System (140) erfassten Gesichtsfeld (141) hat, welches als Referenzpunkt dient und/oder auf dem Untersuchungsobjekt mindestens ein als Referenzpunkt verwendbares Merkmal auf dem Objekt durch das Verfahren (300) erzeugt wird, und bei dem das Erfassen der Lagedaten die Schritte aufweist:
- Erfassen des durch das optische System (140) bereitgestellten ersten und zweiten Halbbildes (112, 114) des zu untersuchenden Objekts (205) durch das Sensorsystem (110);
- Bestimmen einer ersten vertikalen und/oder horizontalen Bildkoordinate (410a, 410b, 420a, 420b) des mindestens einen Referenzpunktes in dem ersten Halbbild (112) und einer zweiten entsprechend vertikalen und/oder horizontalen Bildkoordinate (410a, 410b, 420a, 420b) des mindestens einen Referenzpunktes in dem zweiten Halbbild (114),
- wobei eine etwaige horizontale Bildkoordinate (410a, 410b) bezüglich einer horizontalen Bildachse (440) bestimmt wird, die innerhalb einer Bildebene des stereoskopischen optischen Systems (140) liegt und beide optische Achsen des stereoskopischen optischen Systems (140) schneidet, und
- wobei eine etwaige vertikale Bildkoordinate (420a, 420b) bezüglich einer vertikalen Bildachse (460a, 460b) bestimmt wird, die innerhalb der Bildebene des optischen Systems (140) und senkrecht zur horizontalen Bildachse (440) liegt.

3. Verfahren (300) gemäß Anspruch 2, bei dem das erste und zweite Halbbild (112, 114) des vorbestimmten Referenzobjekts (105) durch das Sensorsystem (110) erfasst werden und bei dem das Justieren des Bildprozessors (130) durch einen Vergleich der auf dem ersten Halbbild (112) abgebildeten mindestens zwei bekannten sichtbaren ReferenzobjektMerkmale (107) mit den auf dem zweiten Halbbild (114) abgebildeten mindestens zwei bekannten sichtbaren Referenzobjekt-Merkmalen (107) erfolgt.

4. Verfahren (300) gemäß Anspruch 3, bei dem das Verfahren (300) neben dem Ermitteln der optischen Verzeichnung ein Ermitteln einer Vergrößerungsdifferenz zwischen dem ersten und zweiten Halbbild (112, 114), oder einer relativen Verschiebung zwischen dem ersten und zweiten Halbbild (112, 114), oder einer Rotation zwischen dem ersten und zweiten Halbbild (112, 114), oder eine Kombination daraus aufweist.

5. Verfahren (300) gemäß mindestens einem der vorhergehenden Ansprüche, bei dem das Einstellen der entsprechenden Verschiebung derart erfolgt, dass durch die geometrische Entzerrung verarbeitete Bildinformationen entsprechend der Lagedaten verschoben werden.

6. Verfahren (300) gemäß mindestens einem der vorhergehenden Ansprüche, bei dem das Einstellen der entsprechenden Verschiebung derart erfolgt, dass entsprechend Lagedaten verschobene Bildinformationen durch die geometrische Entzerrung verarbeitet werden.

7. Verfahren (300) gemäß mindestens einem der vorhergehenden Ansprüche, bei dem weiterhin die Einstellung der geometrischen Entzerrung und/oder die Einstellung der Verschiebung als Justierparameter gespeichert werden.

8. Verfahren (300) gemäß mindestens einem der vorhergehenden Ansprüche, bei dem der mindestens eine Referenzpunkt durch ein Bereitstellen eines auf das zu untersuchende Objekt (205) gerichteten Lichtstrahls (275) gebildet wird.

9. Justiersystem (100, 200) zur Verwendung mit einem optischen System (140), insbesondere mit einem stereoskopischen optischen System (140), zum Erfassen eines zu untersuchenden Objekts (205), aufweisend:
- ein vorbestimmtes Referenzobjekt (105) mit bekannten sichtbaren Referenzobjekt-Merkmalen (107);
- ein Sensorsystem (110), das ausgebildet ist, ein durch das optische System (140) bereitgestelltes Bild oder, bei einem stereoskopischen System ein, erstes und zweites Halbbild (112, 114) eines in einem durch das optische System (140) abzubildenden Gesichtsfeld (141) angeordneten Objekts zu erfassen, wobei das erste (112) und das zweite Halbbild (114) auf einer Abbildungsfläche (115) des Sensorsystems (110) abgebildet werden; und
- ein Datenauswertungs-Modul (120), das mit dem Sensorsystem (110) verbunden ist, und ausgebildet ist, die vom Sensorsystem (110) erfassten Bilddaten (118) zu empfangen und aus den Bilddaten (118) eine Positionsinformation (125) mindestens zweier sichtbarer Referenzobjekt-Merkmale (107) des in dem Gesichtsfeld (141) angeordneten vorbestimmten Referenzobjekts (105) zu bestimmen, und das weiterhin ausgebildet ist, Lagedaten zu bestimmen, welche eine durch mindestens einen Referenzpunkt bestimmte Lageabweichung eines durch das optische System (140) bereitgestellten Bildes des zu untersuchenden Objekts (205) gegenüber erfassten Lagedaten früherer Zeitpunkte zur Ermittlung einer Beschleunigungsinformation des optischen Systems oder bei einem stereoskopischen optischen System (140) einer gegenseitigen Lage eines ersten und zweiten Halbbildes (112, 114) des zu untersuchenden Objekts (205) enthalten; und
- einen Bildprozessor (130), der mit dem Datenauswertungs-Modul (120) verbunden ist und ausgebildet ist, aus der Positionsinformation (125) der mindestens zwei sichtbaren Referenzobjekt-Merkmale (107) des Referenzobjekts (105) eine optische Verzeichnung des Bildes oder des ersten und/oder zweiten Halbbildes (112, 114) zu ermitteln und eine entsprechende geometrische Entzerrung von empfangenen Bilddaten (118) einzustellen, und der weiterhin ausgebildet ist, entsprechend der erfassten Lageabweichung eine entsprechende Verschiebung von empfangenen Bilddaten (118) des Bildes oder des ersten und/oder zweiten Halbbildes (112, 114) einzustellen.

10. Justiersystem (100, 200) gemäß Anspruch 9, das weiterhin ein Speicher-Modul (250) aufweist, das mit dem Bildprozessor (130) verbunden ist und ausgebildet ist, die Einstellung der geometrischen Entzerrung und/oder die Einstellung der Verschiebung als Justierparameter zu speichern.

11. Justiersystem (100, 200) gemäß Anspruch 9 oder 10, bei dem das vorbestimmte Referenzobjekt (105) eine Messtafel ist, die vorbestimmte geometrische Formen in vorbestimmter Weise darstellt.

12. Justiersystem (100, 200) gemäß mindestens einem der Ansprüche 9 bis 11, das weiterhin eine Beleuchtungseinrichtung (270) aufweist, die ausgebildet ist, einen auf das zu untersuchende Objekt (205) gerichteten Lichtstrahl (275) bereitzustellen.

13. Verwendung des Justiersystems (100, 200) gemäß mindestens einem der Ansprüche 9 bis 12 in einem Regelkreis zur dynamischen Kompensation von Dejustierungen eines optischen Systems (140), insbesondere eines stereoskopischen optischen Systems (140), insbesondere eines Stereoendoskops.

14. Medizinisches Gerät, insbesondere medizinisches stereoskopisches Gerät, insbesondere ein Stereoendoskop, das ein Justiersystem (100, 200) gemäß mindestens einem der Ansprüche 9 bis 12 aufweist.

15. Computer-Programm zum Steuern eines Verfahrens (300) gemäß mindestens einem der Ansprüche 1 bis 8 durch einen Computer.

## Claims

1. A method (300) for adjusting an optical system (140), in particular a stereoscopic optical system (140), comprising:
- arranging a predetermined reference object (105) having known visible reference object features (107) in a field of view (141) captured by the optical system (140);
- capturing an image - provided by the optical system (140) - of the predetermined reference object (105) or, in the case of a stereoscopic optical system (140), a first and/or second half-image (112, 114) of the predetermined reference object (105) by means of a sensor system (110) wherein the first (112) and the second half-image (114) are imaged on an imaging area (115) of the sensor system (110);
- determining position information (125) of at least two visible reference object features (107) of the predetermined reference object (105) that are imaged in the image or in the first and/or second half-images (112, 114);
- ascertaining an optical distortion of the image or of the first and/or second half-image (112, 114) from the position information (125) of the at least two visible reference object features (107);
- adjusting an image processor (130), which is at least indirectly connected to the sensor system (110), by setting a geometric rectification in a manner corresponding to the optical distortion ascertained;
wherein the method (300) furthermore comprises the following steps during operation of the optical system (140) for capturing an object to be examined (205):
- arranging the object to be examined (205) in the field of view (141) captured by the optical system (140);
- capturing position data containing a position deviation - determined by means of at least one reference point - of an image - provided by the optical system (140) - of the object to be examined (205) over captured position data from earlier points in time to determine acceleration information of the optical system or, in the case of a stereoscopic optical system (140) of a mutual position of a first and second half-image (112, 114) of the object to be examined (205); and
- adjusting the image processor (130) in a manner corresponding to the captured position deviation by setting a corresponding displacement of the image or of the first and/or second half-image (112, 114) by means of the image processor (130).

2. The method (300) as claimed in claim 1, wherein the optical system (140) is stereoscopic, wherein the object to be examined (205) has at least one visible examination object feature (430a, 430b) in the field of view (141) captured by the optical system (140), which serves as a reference point, and/or on the examination object at least one feature on the object that is useable as a reference point is generated by the method (300), and wherein capturing the position data comprises the following steps:
- capturing the first and second half-images (112, 114) - provided by the optical system (140) - of the object to be examined (205) by means of the sensor system (110);
- determining a first vertical and/or horizontal image coordinate (410a, 410b, 420a, 420b) of the at least one reference point in the first half-image (112) and a second correspondingly vertical and/or horizontal image coordinate (410a, 410b, 420a, 420b) of the at least one reference point in the second half-image (114),
- wherein a possible horizontal image coordinate (410a, 410b) is determined with respect to a horizontal image axis (440) which lies within an image plane of the stereoscopic optical system (140) and intersects both optical axes of the stereoscopic optical system (140), and
- wherein a possible vertical image coordinate (420a, 420b) is determined with respect to a vertical image axis (460a, 460b) which lies within the image plane of the optical system (140) and perpendicularly to the horizontal image axis (440).

3. The method (300) as claimed in claim 2, wherein the first and second half-images (112, 114) of the predetermined reference object (105) are captured by the sensor system (110), and wherein adjusting the image processor (130) is carried out by a comparison of the at least two known visible reference object features (107) imaged on the first half-image (112) with the at least two known visible reference object features (107) imaged on the second half-image (114).

4. The method (300) as claimed in claim 3, wherein the method (300) comprises, besides ascertaining the optical distortion, ascertaining a difference in magnification between the first and second half-images (112, 114), or a relative displacement between the first and second half-images (112, 114), or a rotation between the first and second half-images (112, 114), or a combination thereof.

5. The method (300) as claimed in at least one of the preceding claims, wherein setting the corresponding displacement is carried out in such a way that image information processed by the geometric rectification is displaced in a manner corresponding to the position data.

6. The method (300) as claimed in at least one of the preceding claims, wherein setting the corresponding displacement is carried out in such a way that image information displaced in a manner corresponding to position data is processed by the geometric rectification.

7. The method (300) as claimed in at least one of the preceding claims, wherein furthermore the setting of the geometric rectification and/or the setting of the displacement are/is stored as adjustment parameter(s).

8. The method (300) as claimed in at least one of the preceding claims, wherein the at least one reference point is formed by providing a light beam (275) directed onto the object to be examined (205).

9. An adjustment system (100, 200) for use with an optical system (140), in particular with a stereoscopic optical system (140), for capturing an object to be examined (205), comprising:
- a predetermined reference object (105) having known visible reference object features (107);
- a sensor system (110) configured to capture an image provided by the optical system (140) or, in the case of a stereoscopic system, a first and second half-image (112, 114) of an object arranged in a field of view (141) to be imaged by the optical system (140), wherein the first (112) and the second half image (114) are imaged on an imaging area (115) of the sensor system (110); and
- a data evaluation module (120), which is connected to the sensor system (110) and is configured to receive the image data (118) captured by the sensor system (110) and to determine from the image data (118) position information (125) of at least two visible reference object features (107) of the predetermined reference object (105) arranged in the field of view (141), and which is furthermore configured to determine position data containing a position deviation - determined by means of at least one reference point - of an image - provided by the optical system (140) - of the object to be examined (205) over captured position data from earlier points in time to determine acceleration information of the optical system or, in the case of a stereoscopic optical system (140) of a mutual position, of a first and second half-image (112, 114) of the object to be examined (205); and
- an image processor (130), which is connected to the data evaluation module (120) and is configured to ascertain an optical distortion of the image or of the first and/or second half-image (112, 114) from the position information (125) of the at least two visible reference object features (107) of the reference object (105) and to set a corresponding geometric rectification of received image data (118), and which is furthermore configured to set a corresponding displacement of received image data (118) of the image or of the first and/or second half-image (112, 114) in a manner corresponding to the position deviation captured.

10. The adjustment system (100, 200) as claimed in claim 9, which furthermore comprises a storage module (250), which is connected to the image processor (130) and is configured to store the setting of the geometric rectification and/or the setting of the displacement as adjustment parameters).

11. The adjustment system (100, 200) as claimed in claim 9 or 10, wherein the predetermined reference object (105) is a measurement chart that represents predetermined geometric shapes in a predetermined manner.

12. The adjustment system (100, 200) as claimed in at least one of claims 9 to 11, which furthermore comprises an illumination device (270) configured to provide a light beam (275) that is directed onto the object to be examined (205).

13. The use of the adjustment system (100, 200) as claimed in at least one of claims 9 to 12 in a control loop for the dynamic compensation of misadjustments of an optical system (140), in particular of a stereoscopic optical system (140), in particular of a stereo endoscope.

14. A medical apparatus, in particular a medical stereoscopic apparatus, in particular a stereo endoscope, which comprises an adjustment system (100, 200) as claimed in at least one of claims 9 to 12.

15. A computer program for controlling a method (300) as claimed in at least one of claims 1 to 8 by means of a computer.

## Revendications

1. Procédé (300) d'alignement d'un système (140) optique, notamment d'un système (140) optique stéréoscopique, comportant :
- la mise d'un objet (105) de référence déterminée à l'avance ayant des caractéristiques (107) connues visibles dans un champ (141) de vision pris par le système (140) optique ;
- la détection d'une image, donnée par le système (140) optique, de l'objet (105) de référence déterminée à l'avance ou pour un système (140) optique stéréoscopique d'une première et/ou d'une deuxième demi-image (112, 114) de l'objet (105) de référence déterminée à l'avance par un système (110) de capteur, dans lequel la première (112) et la deuxième demi-image (114) sont reproduites sur une surface (115) de reproduction du système (110) de capteur ;
- la détermination d'une information (125) de position au moyen de deux caractéristiques (107) visibles de l'objet (105) de référence déterminée à l'avance reproduites dans l'image ou dans la première et/ou dans la deuxième demi-image (112, 114) ;
- la détermination d'une distorsion optique de l'image ou de la première et/ou de la deuxième demi-image (112, 114) à partir de l'information (125) de position des au moins deux caractéristiques (107) visibles de l'objet de référence ;
- l'alignement d'un processeur (130) d'images, relié au moins indirectement au système (110) de capteur, par réglage d'une compensation géométrique de distorsion correspondant à la distorsion optique déterminée ;
dans lequel le procédé a en outre pendant un fonctionnement du système (140) optique les stades suivants pour la détection d'un objet (205) à examiner
- la mise de l'objet (205) à examiner dans le champ (141) de vision pris par le système (140) optique ;
- la détection de données de position, qui contiennent un écart de position, déterminé par au moins un point de référence, d'une image donnée par le système (140) optique de l'objet (205) à examiner par rapport à des données de position détectées à des instants antérieurs afin de déterminer une information d'accélération du système optique ou, pour un système (140) optique stéréoscopique, une position mutuelle d'une première et d'une deuxième demi-image (112, 114) de l'objet (205) à examiner ; et
- l'alignement du processeur (130) d'images conformément à l'écart de position détecté par réglage d'un décalage correspondant de l'image ou de la première et/ou de la deuxième demi-image (112, 114) par le processeur (130) d'images.

2. Procédé (300) suivant la revendication 1, dans lequel le système (140) optique est stéréoscopique, dans lequel l'objet (205) à examiner a dans le champ (141) de vision pris par le système (140) optique au moins une caractéristique (430a, 430b) visible, qui sert de point de référence, et/ou sur l'objet à examiner est produite par le procédé (300) au moins une caractéristique sur l'objet pouvant être utilisée comme point de référence, et dans lequel la détection des données de position comporte les stades :
- la détection par le système (110) de capteur des première et deuxième demi-images (112, 114) de l'objet (105) à examiner données par le système (140) optique ;
- la détermination d'une première coordonnée (410a, 410b, 420a, 420b) d'images verticale et/ou horizontale du au moins un point de référence dans la première demi-image (112) et d'une deuxième coordonnée (410a, 410b, 420a, 420b) d'image verticale et/ou horizontale correspondante du au moins un point de référence dans la deuxième demi-image (114) ;
- dans lequel on détermine une coordonnée (410a, 410b) d'image éventuellement horizontale par rapport à un axe (440) horizontal d'image, qui est dans un plan d'image du système (140) optique stéréoscopique et qui coupe les deux axes optiques du système (140) optique stéréoscopique, et
- dans lequel on détermine une coordonnée (420a, 420b) d'image éventuellement verticale par rapport à un axe (460a, 460b) vertical d'image, qui est dans le plan d'image du système (140) optique et perpendiculaire à l'axe (440) horizontal d'image.

3. Procédé (300) suivant la revendication 2, dans lequel on détecte par le système (110) de capteur la première et la deuxième demi-image (112, 114) de l'objet (105) de référence déterminé à l'avance et dans lequel on effectue l'alignement du processeur (130) d'images en comparant les au moins deux caractéristiques (107) visibles connues de l'objet de référence reproduites sur la première demi-image (112) aux au moins deux caractéristiques (107) visibles connues de l'objet de référence reproduites sur la deuxième demi-image (114).

4. Procédé (300) suivant la revendication 3, dans lequel le procédé comporte outre la détermination de la distorsion optique, une détermination d'une différence d'agrandissement entre la première et la deuxième demi-image (112, 114) ou un décalage relatif entre la première et la deuxième demi-image (112, 114) ou une rotation entre la première et la deuxième demi-image (112, 114) ou une combinaison de ceux-ci.

5. Procédé (300) suivant au mois l'une des revendications précédentes, dans lequel le réglage du décalage correspondant s'effectue en décalant conformément aux données de position des informations d'image traitées par la correction géométrique de distorsion.

6. Procédé (300) suivant au moins l'une des revendications précédentes, dans lequel le réglage du décalage correspondant s'effectue en traitant par la correction géométrique de distorsion des informations d'image décalées conformément aux données de position.

7. Procédé (300) suivant au moins l'une des revendications précédentes, dans lequel en outre on met en mémoire comme paramètre d'alignement le réglage de la correction géométrique de distorsion et/ou le réglage du décalage.

8. Procédé (300) suivant au moins l'une des revendications précédentes, dans lequel on forme le au moins un point de référence en disposant d'un faisceau (275) lumineux dirigé sur l'objet (205) à examiner.

9. Système (100, 200) d'alignement à utiliser avec un système (140) optique, notamment avec un système (140) optique stéréoscopique, pour la détection d'un objet (205) à examiner, comportant :
- un objet (105) de référence déterminée à l'avance ayant des caractéristique (107) connues visibles ;
- un système (110) de capteur, qui est constitué pour détecter une image donnée par le système (140) optique ou, si le système est stéréoscopique, une première et une deuxième demi-image (112, 114) d'un objet disposé dans un champ (141) de vision à reproduire par le système (140) optique, la première (112) et la deuxième demi-images (114) étant reproduites sur une surface (115) de reproduction du système (110) de capteur ; et
- un module (120) d'exploitation de données, qui est relié au système (110) de capteur et qui est constitué pour recevoir des données (118) d'image détectées par le système (110) de capteur et pour déterminer à partir des données (118) d'image une information (125) de position d'au moins deux caractéristiques (107) visibles de l'objet (105) de référence déterminé à l'avance disposé dans le champ (141) de vision, et qui est constitué en outre pour déterminer des données de position, qui contiennent un écart de position, déterminé par au moins un point de référence, d'une image, donnée par le système (140) optique, de l'objet (205) à examiner par rapport à des données de position détectées à des instants antérieurs, afin de déterminer une information d'accélération du système optique ou, pour un système (140) optique stéréoscopique, une position mutuelle d'une première et d'une deuxième demi-image (112, 114) de l'objet (205) à examiner ; et
- un processeur (130) d'images, qui est relié au module (120) d'exploitation de données et qui est constitué pour déterminer, à partir de l'information (125) de position des au moins deux caractéristiques (107) visibles de l'objet (105) de référence, une distorsion optique de l'image ou de la première et/ou de la deuxième demi-image (112, 114) et pour régler une correction géométrique de distorsion correspondante des données (118) d'image reçues et qui est constitué en outre pour régler, conformément à l'écart de position détectée, un décalage correspondant des données (118) d'image reçues de l'image ou de la première et/ou de la deuxième demi-image (112, 114).

10. Système (100, 200) d'alignement suivant la revendication 9, qui a en outre un module (250) de mémoire, qui est relié au processeur (130) d'image et qui est constitué pour mettre en mémoire comme paramètre d'alignement le réglage de la correction géométrique de distorsion et/ou le réglage du décalage.

11. Système (100, 200) d'alignement suivant la revendication 9 ou 10, dans lequel l'objet (105) de référence déterminé à l'avance est un panneau de mesure, qui représente d'une façon déterminée à l'avance les formes géométriques déterminées à l'avance.

12. Système (100, 200) d'alignement suivant au moins l'une des revendications 9 à 11, qui a en outre un dispositif (270) d'éclairage constitué pour disposer d'un faisceau (275) lumineux dirigé sur l'objet (205) à examiner.

13. Utilisation du système (100, 200) d'alignement suivant au moins l'une des revendications 9 à 12, dans un circuit de réglage pour la compensation dynamique de désalignement d'un système (140) optique, notamment d'un système (140) optique stéréoscopique, notamment d'un stéréoendoscope.

14. Appareil médical, notamment appareil médical stéréoscopique, notamment stéréoendoscope, qui a un système (100, 200) d'alignement suivant au moins l'une des revendications 9 à 12.

15. Programme d'ordinateur pour la commande d'un procédé (300) suivant au moins l'une des revendications 1 à 8, par un ordinateur.
